# EUROPEAN PATENT APPLICATION

(11) **EP 4 516 328 A1**
(43) Date of publication of application: **05.03.2025**
(21) Application number: 24196580.5
(22) Date of filing: 27.08.2024
(51) Int. Cl.: A61L 27/04, A61L 27/58

(54) **IMPLANT FOR COVERING BONE DEFECTS**

(30) Priority: 28.08.2023 CN 202322306643 U; 28.09.2023 US 202363586202 P; 01.03.2024 US 202463559923 P
(71) Applicant: Botiss Biomaterials GmbH, 15806 Zossen (DE)
(72) Inventor: Bielenstein, Oliver, 14193 Berlin (DE); Tadic, Dr. Drazen, 14129 Berlin (DE)
(74) Representative: Friderichs, Gunther

(57) **Abstract**

The disclosure relates to a method for producing an implant for covering bone defects, wherein the implant consists of a magnesium or magnesium alloy film, comprising the steps: Providing a magnesium or magnesium alloy film;

Removing a layer of material on both sides of the magnesium or magnesium alloy film by grinding.

## Description

### Technical Field

The disclosure relates to a bioresorbable implant for covering bone defects. In particular, the disclosure relates to an implant for covering bone defects in the jaw region.

### Background

Document WO 2016/134797 A1 (Botiss Biomaterials GmbH) discloses a bioresorbable implant for covering bone defects which consists of a magnesium film. The magnesium film separates soft tissue from bone material very effectively in the initial phase after insertion. The magnesium film corrodes after insertion. The released magnesium may further improve the formation of natural bone tissue.

### Summery

It is an object of the invention to provide a method for producing an implant with improved corrosion properties. In particular, a uniform corrosion over the entire surface shall be provided.

Preferred embodiments and refinements of the invention will be apparent from the subject-matter of the dependent claims, the description, and the drawings.

The disclosure relates to a method for producing an implant for covering bone defects, wherein the implant consists of a magnesium or magnesium alloy film.

The method comprises the steps:
Providing a magnesium or magnesium alloy film;
Removing a layer of material on both sides of the magnesium or magnesium alloy film by mechanical means like grinding or polishing.

The inventors found out that the removal and/or purification of a surface layer results in an implant with improved corrosion properties.

Not being bound on the theory, it is suspected that inhomogeneous areas and impurities, which result from the production process of the film, are removed. Removing material from the surface eliminates any impurities introduced during down-stream production processes like rolling or drawing. These impurities may have a negative effect on biocompatibility and may most likely result in local corrosion due to electrochemical reactions, for example, from embedded metallic impurities.

This applies, in particular, for a film which is produced by a rolling or drawing process. Due to the rolling or drawing, the surface may comprise areas with a varying microstructure, in particular grain size, with a varying hardness or embedded foreign materials from the rollers and/or processing aids like lubricants.

According to an embodiment of the invention, the magnesium or magnesium alloy film is etched after the step of removing a layer of material on both sides of the magnesium or magnesium alloy film by grinding or polishing. It has been found that the etching process is markedly more efficient and effective if the sheets have been polished or ground prior to etching.

Preferably, the etching process results in removing a second layer of material.

The two steps, grinding and then etching, result in a surface with sophisticated properties against a corrosive attack in the initial phase after inserting the implant.

The step of etching may be performed in an acid bath with may comprise acetic acid and/or nitric acid.

In order to adjust the etching rate, an organic liquid can be added to the etching bath, in particular in an amount of 30 - 80 weight% of the etching bath. In particular, glycerin can be used as the organic liquid.

According to an embodiment of the invention, the thickness of the magnesium alloy film is reduced by 3 to 60 %, preferably by 5 to 50 % by grinding.

In further, the thickness of the layer of magnesium or magnesium alloy can be reduced by 3 to 50 %, preferably by 5 to 30 % by etching.

Preferably, a rolled magnesium or magnesium alloy film is provided.

The thickness of the magnesium or magnesium alloy film can be reduced on each side by a minimum of 10 µm, preferably by a minimum of 30 µm with the step of grinding.

The thickness of the magnesium or magnesium alloy film is further reduced on each side by 5-30 µm, preferably by 10 - 15 µm, with the step of etching.

According to an embodiment of the invention, the magnesium or magnesium alloy film is cut or shaped between the step of grinding and the step of etching. Accordingly, the final surface structure is provided by etching after the implant is already cut and/or shaped. Hence, deviations in the surface structure due to the cutting or shaping process are reduced.

The implant can have a thickness between 100 and 200 µm.

Preferably, the magnesium or magnesium alloy film is closed.

According to another embodiment, the film is perforated. A perforation enables to bend the implant to a desired complex 3-dimensional shape in a sophisticated manner. The perforated implant is used preferably in combination with another second membrane which is placed onto or below the implant. The implant holds the second membrane in a desired shape.

The perforated film is preferably thicker, e.g. 0.5 - 1 mm.

The perforations can be embodied as round holes, curves, in particular with the shape of a boomerang etc.

The outer contour as well as the perforations can be applied e.g. by laser cutting, water jet cutting or by punching.

Preferably, the perforations are distributed in a regular manner.

Preferably, the perforations are applied after the step of grinding and/or before the step of etching.

The homogeneity (or smoothness) of the surface is a critical parameter for the performance of the implant. The more homogenous the surface is, the lower is the risk of bacterial adhesion and the more even will be the corrosive attack. Local corrosion spots result in undesirable holes in the membrane annihilating the desired barrier function separating soft and bone tissue.

The Material Ratio curve (also called Abbott-Firestone curve or Bearing Area Curve) according to DIN EN ISO 21920 - Parts 1-3: 2021-12 and ISO 25178-2:2021 are a method to describe the functional parameters of a surface. In short, the material ratio expresses which percentage of an intersecting plane or line parallel to the mean plane of the surface is material. At the highest peak the Material Ratio is 0% as no material is intersected and is 100% at the lowest valley as the intersecting plane cuts completely through material.

The Material Ratio curve can be divided into a peak, core and valley zone. The more material is located in the core zone, the more homogenous the surface is. The portion lying in the peak or valley zone should be as small as possible. For the given application, the thickness of the core zone should not exceed 1% - 2% of the total thickness of the implant, corresponding to +/- 1 µm to avoid local break-through corrosion.

In particular, the Material Ratio in a core zone of +/- 1 µm as determined from the Abbott-Firestone curve is greater than 97%, in particular 97-99 %.

It had been found out that the Material Ratio is a parameter, which correlates with a uniform corrosion of the implant.

In further, the inventors discovered that with these parameters of the Abott-Firestone curve, also implants with a thickness below 300 µm can be provided, which provide a barrier function for at least two weeks after implantation. Implants below 300 µm can be bend more easily and are less likely to crack during shaping.

According to a further aspect of the disclosure, the surface of the implant has a reduced valley depth Rvk of less than 0.6 µm.

The reduced valley depth is determined according to DIN EN ISO 21920 - Parts 1-3: 2021-12 and ISO 25178 - Parts 1-3.

Preferably, the film has a reduced valley depth Rvk between 0.4 and 0.5 µm.

Preferably, the film has a maximum surface roughness Rt of less than 3 µm. The maximum surface roughness is defined the vertical difference between the deepest groove and the highest peak of the total measuring section.

The maximum surface roughness Rt is measured according to DIN EN ISO 21920 - Parts 1-3: 2021-12 and ISO 25178 - Parts 1-3.

Preferably, the film has a maximum surface roughness Rt between 2 and 3 µm.

According to a further aspect of the disclosure, the film has a root mean square gradient Sdq of less than 5°. The root mean square gradient Sdq is a measurement of the slopes which comprise the surface.

The root mean square gradient Sdq is measured according to DIN EN ISO 21920 - Parts 1-3: 2021-12 and ISO 25178 - Parts 1-3.

Preferably, the film has a root mean square gradient Sdq of 3.5° to 5°.

The film may have a surface roughness Rt between 2 and 3 µm and/or a surface roughness Ra between 0.2 and 0.3 µm and/or a maximum profile valley depth Rv between 0.8 and 1.2 µm. Preferably, the implant is curved or pre-shaped to match a specific anatomic shape. In particular, the curvature is adapted to the curvature of the jaw area, where the implant is inserted.

According to an embodiment, the implant is placed in a package and terminally sterilized by gamma irradiation. In particular, the package is embodied as a tear-open package.

The package can be flooded by an inert gas prior to sealing.

Preferably, the package is gas-tight. The package may consist of a foil which comprises a barrier layer, e.g. aluminum or poly-siloxane.

According to an embodiment, a profile of the Material Ratio Curve has a maximum positive deviation of less than + 1.7 µm and a maximum negative deviation of less than - 2.0 µm, respective the profile of the relative Material Ratio Curve ranges from - 2.0 µm to + 1.7 µm. The relative Material Ratio Curve can be determined according to DIN EN ISO 21920 - Parts 1-3: 2021-12 and ISO 25178 - Parts 1-3.

The relative Material Ratio Curve describes the percentage of material which is traversed by a cut at a certain level located with respect to the highest point on the profile.

In particular, the profile of the relative Material Ratio Curve has a maximum positive deviation of less than + 1.5 µm and a maximum negative deviation of less than - 1.8 µm, respectively the relative Material Ratio Curve ranges from - 1.8 µm to + 1.5 µm (according to DIN EN ISO 21920 - Parts 1-3: 2021-12 and ISO 25178 - Parts 1-3).

According to an embodiment, the film-like implant is circular or ovoid, four-sided, hour-glass shaped or otherwise pre-contoured to match a specific anatomic shape.

The film-like implant might be embodied flat, "L"-like or "U"-like pre-shaped.

The film-like implant may comprise an opening for another implant, especially a tooth implant.

According to an embodiment, the magnesium or magnesium alloy film is cleaned and passivated after the step of etching.

The step of passivation may be performed by subjecting the foil to oxygen, e.g. by drying the cleaned implant in a clean room for a sufficient amount of time.

The passivation results in a protective magnesium oxide layer on the surface. This avoids the formation of magnesium containing reaction products formed from the material of the package and the magnesium of the implant.

The package can be embodied as gas-tight foil bag. To archive gas-tight properties, the foil of the foil bag may comprise a metal or a metal oxide layer.

The disclosure further relates to a method for producing an implant for covering bone defects, wherein the implant consists of a magnesium or magnesium alloy film.

The method comprises the steps:
Providing a rolled magnesium or magnesium alloy film;
Removing a layer of material on both sides of the magnesium or magnesium alloy film

As already discussed above, inhomogeneities or impurities due to the rolling process are removed. The step of removing a layer of material is preferably performed by grinding and etching.

According to a further aspect, the disclosure relates to method for producing an implant for covering bone defects.

In particular, the method comprises at least one of above-mentioned steps.

The implant consists of a magnesium or magnesium alloy film, which is providing as a rolled magnesium or magnesium alloy film;
According to the disclosure, a surface of the magnesium or magnesium alloy film is laser treated. In particular. The, the surface of the magnesium or magnesium alloy is laser polished. When laser radiation is used to polish the surface, a thin surface layer of the workpiece is remelted and the surface smoothed due to interfacial tension.

Preferably, the surface is laser treated before an etching step.

It had been found out that inhomogeneities of the etched surface can reduced. Not being bound to this theory, the inventors suspect that organic inclusions on or in the surface are removed by the step of laser treatment. These inclusions might be burned or vaporized.

In particular, organic inclusions seem to strengthen the surface against the corrosive attack of the etching step. This may result in spots, in particular being embodied as elevations, on the surface.

According to a further aspect, the disclosure relates to a method for producing an implant for covering bone defects. In particular, the method comprises at least one of above-mentioned steps. The implant consists of a magnesium or magnesium alloy film, preferably a rolled film. According to the disclosure, a surface of the magnesium or magnesium alloy film is purified by mechanical means or by physical means. Preferably, organic impurities, in particular aliphatic carbon compounds, are removed.

This can be performed, as described above, by grinding or polishing. Also physical means, in particular plasma treatment or remelting the surface by laser treatment, in particular laser polishing.

According to a further embodiment of the invention, the magnesium or magnesium alloy film is tempered. In particular, the magnesium or magnesium alloy film is tempered between 150 and 250 °C. Tempering homogenizes the crystalline structure of the implant which makes it easier to bend.

The disclosure also relates to an implant being produced with the method as described above. The disclosure also relates to an implant, being embodied as a magnesium or magnesium alloy film, comprising a perforation which extends over the entire area of the magnesium or magnesium alloy film. Hence, the implant is perforated in a central area thereof also.

As described above, the perforated film can have a thickness of 0.5 - 1 mm. The perforated implant can serve as a 3-dimentional support structure for another membrane.

The disclosure further relates to a method for inserting a dental implant, said method comprises the steps of
- inserting a magnesium or magnesium alloy film between bone and soft tissue at a sidewall of defect side of the bone;
- filling the defect side with bone substitution material;
- inserting the dental implant into said defect side.

The magnesium or magnesium alloy film is preferably embodied as an implant as described above.

According to this embodiment, the film preferably has a thickness of 120 - 200 µm.

The dental implant can be embodied as a screw for an artificial tooth.

It had been found out that the magnesium or magnesium alloy film stabilizes the sidewall of the defect side very effectively. In particular, deformations of the sidewall due to pressure from the tongue, can be avoided.

The defect side can be embodied an alveolar channel of a lost or extracted tooth. The foil can be cut and bended to a form-stable three-dimensional shape, which fits to the shape of the sidewall. In particular, the foil can be inserted at the buccal and/or palatal wall of the defect side.

Preferably, the magnesium or magnesium alloy film is clamped between soft tissue and bone. Hence, the use of fasteners as pins and screws can be avoided. If needed or desired the film may further be fixed by the use of magnesium or magnesium alloy screws or pins. According to an embodiment of the invention, the magnesium or magnesium alloy film is folded onto the defect side. According to this embodiment of the invention, also the top side of the defect side, which is filled with the bone substitution material, is closed by the foil. The folding edge stabilizes the three-dimensional shape of the foil.

The implant consisting of a foil can be used to treat single or double wall defect after tooth extraction, where there is formed a defect side with insufficient bone and support for tooth implant.

The foil enables a quick and easy adjustment of a scaffold according to the patient's specific anatomy and provides sufficient strength, particularly at the rim, to withstand tongue pressure/forces during chewing until consolidation of bone graft.

The edge of the foil can be folded, resulting in a smooth and rounded rim to protect gingiva when covering defects. Single or multiple bending/folding of rim results in a smooth and stiff rim protecting the gingiva and providing strength to withstand in vivo forces.

In contrary to polymer materials, the magnesium or magnesium alloy film Material needs not to be heated for bending. Time consuming heating and re-heating is avoided.

The disclosure can also be defined by an implant or by a method for producing an implant according to following items:
1. Implant for covering bone defects, wherein the implant consists of a magnesium or magnesium alloy film, wherein the magnesium or magnesium alloy film has a reduced valley depth Rvk of less than 0.6 µm (according to DIN EN ISO 21920 - Parts 1-3: 2021-12 and ISO 25178 - Parts 1-3).
2. Implant for covering bone defects, in particular according to item 1, wherein the implant consists of a magnesium or magnesium alloy film, wherein the magnesium or magnesium alloy film has a maximum surface roughness Rt of less than 3 µm (according to DIN EN ISO 21920 - Parts 1-3: 2021-12 and ISO 25178 - Parts 1-3).
3. Implant for covering bone defects, in particular according to item 1, wherein the film has a root mean square gradient Sdq of less than 5° (according to DIN EN ISO 21920 - Parts 1-3: 2021-12 and ISO 25178 - Parts 1-3).
4. Implant according to any of the preceding items, wherein the film has a reduced valley depth Rvk between 0.4 and 0.5 µm.
5. Implant according to any of the preceding items, wherein the film has a maximum surface roughness Rt between 2 and 3 µm.
6. Implant according to any of the preceding items, wherein the film has a root mean square gradient Sdq of 3.5° to 5°.
7. Implant according to any of the preceding items, wherein the film has a surface roughness Rt between 2 and 3 µm.
8. Implant according to any of the preceding items, wherein the film has a surface roughness Ra between 0.2 and 0.3 µm.
9. Implant according to any of the preceding items, wherein the film has a maximum profile valley depth Rv between 0.8 and 1.2 µm.
10. Implant according to any of the preceding items, wherein the film is grinded or polished and etched, preferably thereafter.
11. Implant according to any of the preceding items, wherein the film has a thickness between 100 and 200 µm.
12. Implant according to any of the preceding items, wherein the implant is curved or pre-shaped to match a specific anatomic shape.
13. Implant according to any of the preceding items, wherein the implant is placed in a package and terminally sterilized by gamma irradiation.
14. Implant according to any of the preceding items, wherein the package is flooded by an inert gas prior to sealing.
15. Implant according to any of the preceding items, wherein the package is gas-tight.
16. Implant according to any of the preceding items, wherein the profile of the relative Material Ratio Curve ranges from - 2.0 µm to + 1.7 µm (according to DIN EN ISO 21920 - Parts 1-3: 2021-12 and ISO 25178 - Parts 1-3).
17. Implant according to any of the preceding items, wherein the profile of the relative Material Ratio Curve ranges from - 1.8 µm to + 1.5 µm (according to DIN EN ISO 21920 - Parts 1-3: 2021-12 and ISO 25178 - Parts 1-3).
18. Implant according to any of the preceding items, wherein the material area ratio in a core zone of +/- 1 µm as determined from the Abbott-Firestone curve (material ratio curve) according to DIN EN ISO 21920 - Parts 1-3: 2021-12 and ISO 25178-2:2021 is greater than 95%.
19. Implant according to any of the preceding items, wherein the material area ratio in a core zone of +/- 1 µm as determined from the Abbott-Firestone curve (material ratio curve) according to DIN EN ISO 21920 - Parts 1-3: 2021-12 and ISO 25178-2:2021 is greater than 97%.
20. Implant according to any of the preceding items, wherein the outer contour of the film-like implant is circular or ovoid, four-sided, hour-glass shaped to match a specific anatomic shape.
21. Implant according to any of the preceding items, wherein the implant is three-dimensionally pre-shaped to match a specific anatomic shape.
22. Implant according to any of the preceding items, wherein the implant is "L"-like or "U"-like pre-shaped.
23. Implant according to any of the preceding items, wherein the implant has an opening for another implant, especially for a tooth implant.
24. A method for producing an implant for covering bone defects, wherein the implant consists of a magnesium or magnesium alloy film, comprising the steps:
   providing a magnesium or magnesium alloy film;
   removing a layer of material on both sides of the magnesium or magnesium alloy film,
   in particular by grinding or polishing.
25. The method according to the preceding item, further comprising: etching the magnesium or magnesium alloy film after removing the layer of material on both sides of the magnesium or magnesium alloy film.
26. The method according to any of the preceding items, wherein a thickness of the magnesium alloy film is reduced by 3 to 60 % by grinding.
27. The method according to any of the preceding items, wherein a remaining thickness of the magnesium or magnesium alloy film after the grinding is reduced by 3 to 50 % by the etching.
28. The method according to any of the preceding items, wherein the magnesium or magnesium alloy film is a rolled magnesium or magnesium alloy film.
29. The method according to any of the preceding items, wherein a thickness of the magnesium or magnesium alloy film is reduced by a minimum of 10 µm on each side, with the step of grinding.
30. The method according to any of the preceding items, wherein a remaining thickness of the magnesium or magnesium alloy film after grinding is further reduced on each side by 5 - 30 µm with the step of etching.
31. The method according to any of the preceding items, further comprising cutting or shaping the magnesium or magnesium alloy film between the grinding or polishing step and the etching step.
32. The method according to any of the preceding items, wherein a magnesium or magnesium alloy film is provided with a material area ratio in a core zone of +/- 1 µm as determined from the Abbott-Firestone curve which is greater than 95%.
33. The method according to any of the preceding items, wherein a magnesium or magnesium alloy film is provided with a reduced valley depth Rvk of less than 0.6 µm.
34. The method according to any of the preceding items, wherein a magnesium or magnesium alloy film is provided with a maximum surface roughness Rt of less than 3 µm.
35. The method according to any of the preceding items, wherein a magnesium or magnesium alloy film is provided with film has a root mean square gradient Sdq of less than 5°.
36. The method according to any of the preceding items, wherein the magnesium or magnesium alloy film has a thickness between 100 and 200 µm.
37. The method according to any of the preceding items, further comprising: shaping the magnesium or magnesium alloy film to form a curved or pre-shaped implant after the step of grinding.
38. The method according to any of the preceding items, further comprising: placing the implant in a package and terminally sterilizing the implant by gamma irradiation.
39. The method according to any of the preceding items, further comprising: flooding the package by an inert gas and thereafter sealing the package.
40. The method according to any of the preceding items, wherein the package is gas-tight.
41. The method according to any of the preceding items, further comprising: shaping the magnesium or magnesium alloy film to form an "L"-shaped or "U"-shaped pre-shaped implant.
42. The method according to any of the preceding items, further comprising: punching or cutting an opening for a further implant into the magnesium or magnesium alloy film.
43. The method according to the preceding item, wherein the further implant is a tooth implant.
44. The method according to any of the preceding items, further comprising: cleaning and passivating the magnesium or magnesium alloy film after the etching.
45. Method for producing an implant for covering bone defects, in particular according to any of the preceding items, wherein the implant consists of a magnesium or magnesium alloy film, comprising:
   providing a rolled magnesium or magnesium alloy film;
   laser treating a surface of the magnesium or magnesium alloy film.
46. The method according to the preceding item, characterized in that the surface of the magnesium or magnesium alloy is laser-polished.
47. The method according to any of the preceding items, wherein the surface is grinded and/or polished after the step of laser-treating.
48. A Method for producing an implant for covering bone defects, in particular according to any of the preceding items, wherein the implant consists of a magnesium or magnesium alloy film, comprising the step of purifying a surface of the magnesium or magnesium alloy film by mechanical means or by physical means.
49. A method for producing an implant for covering bone defects, wherein the implant consists of a magnesium or magnesium alloy film, comprising:
   providing a rolled magnesium or magnesium alloy film;
   removing a layer of material on both sides of the magnesium or magnesium alloy film
50. An implant, being produced with the method according to any of the preceding items.
51. An implant, being embodied as a magnesium or magnesium alloy film, comprising a perforation which extends over the entire area of the magnesium or magnesium alloy film.
52. An implant, being embodied as a magnesium or magnesium alloy film, wherein said implant is embodied as a convex bulge consisting of the magnesium or magnesium alloy film.
53. A method for inserting a dental implant, said method comprises the steps of
   - inserting a magnesium or magnesium alloy film between bone and soft tissue at a sidewall of a defect side of the bone;
   - filling the defect side with bone substitution material;
   - inserting the dental implant into said defect side.
54. The method of any of the preceding items, wherein the magnesium or magnesium alloy film is clamped between soft tissue and bone.
55. The method of any of the preceding items, wherein the magnesium or magnesium alloy film is folded over the defect side.

### Brief Description of the Drawings

The subject matter of the invention will be now explained in more detail with reference to Fig. 1 - Fig. 22b.
Fig. 1 is a flowchart of a method of producing an implant according to an embodiment of the disclosure.
Fig. 2 is a schematic illustration of the step of grinding.
Fig. 3 shows how the implants are further processed to be etched and cleaned.
Fig. 4 - Fig. 7 show various embodiments of an implant.
Fig. 8 - Fig. 10 are cross sections of the various embodiments of an implant.

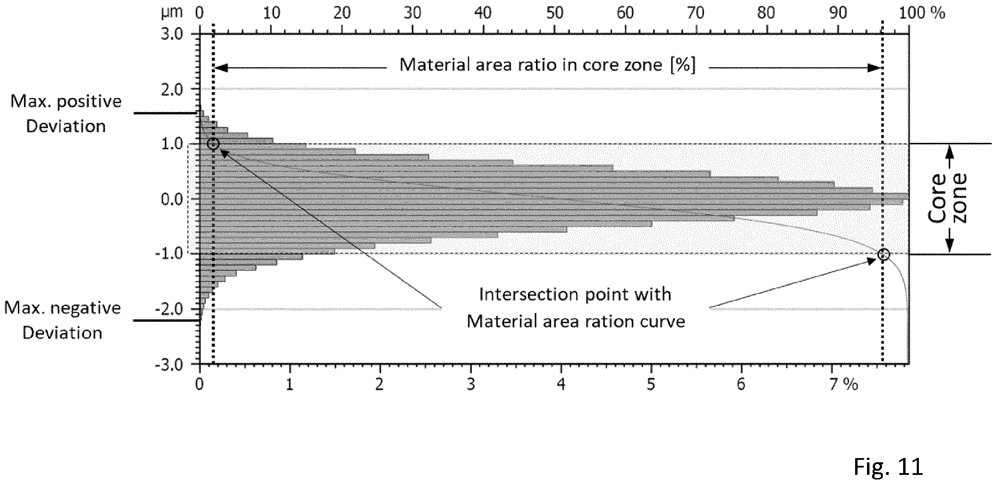

With reference to Fig. 11, the relative material ratio curve of the surface of the implant shall be explained in more detail.

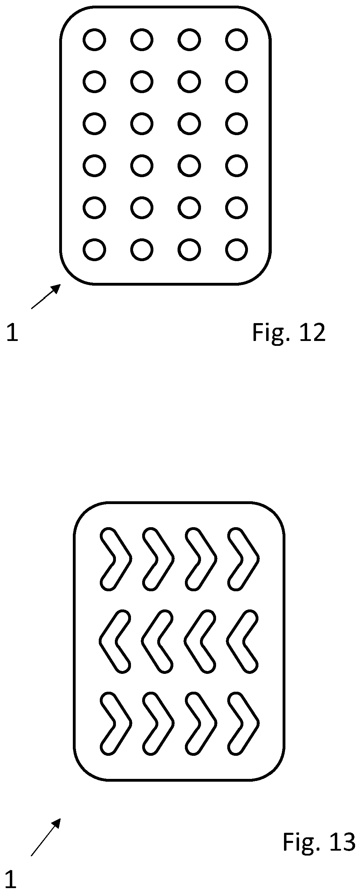

Fig. 12 and 13 show alternative embodiments of an implant with a perforation.

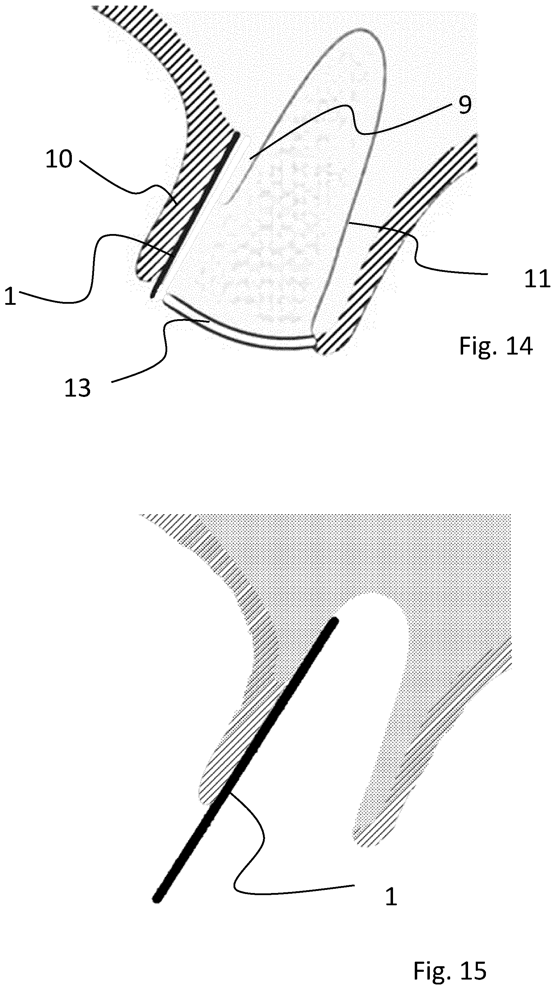

With reference to Fig. 14 - Fig. 17, embodiments of a method for inserting a dental implant are explained.

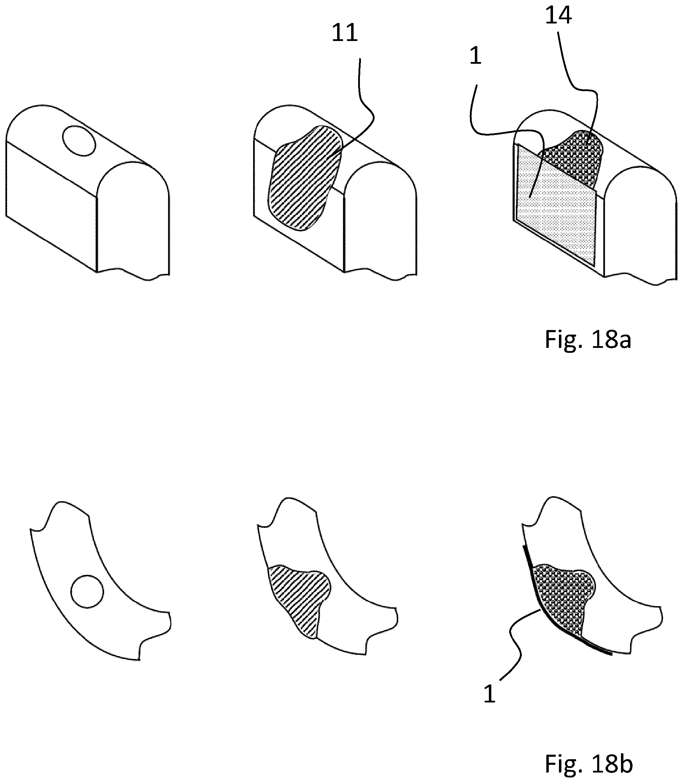

Fig. 18a and 18b illustrate the use of a magnesium or magnesium alloy film for single wall defects.

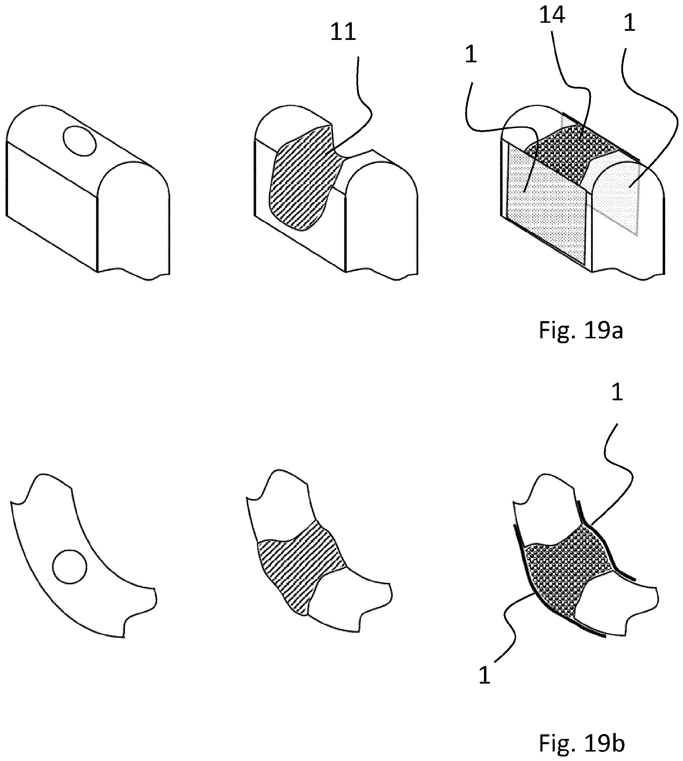

Fig. 19a and 19b illustrate the use of a magnesium or magnesium alloy film for double wall defects.

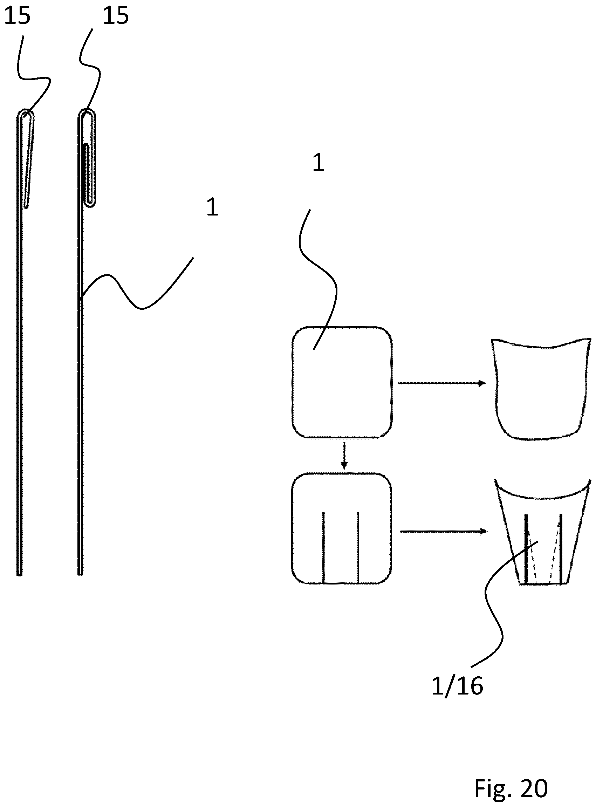

Fig. 20 shows an embodiment of a magnesium or magnesium alloy implant which is folded to a convex bulge.

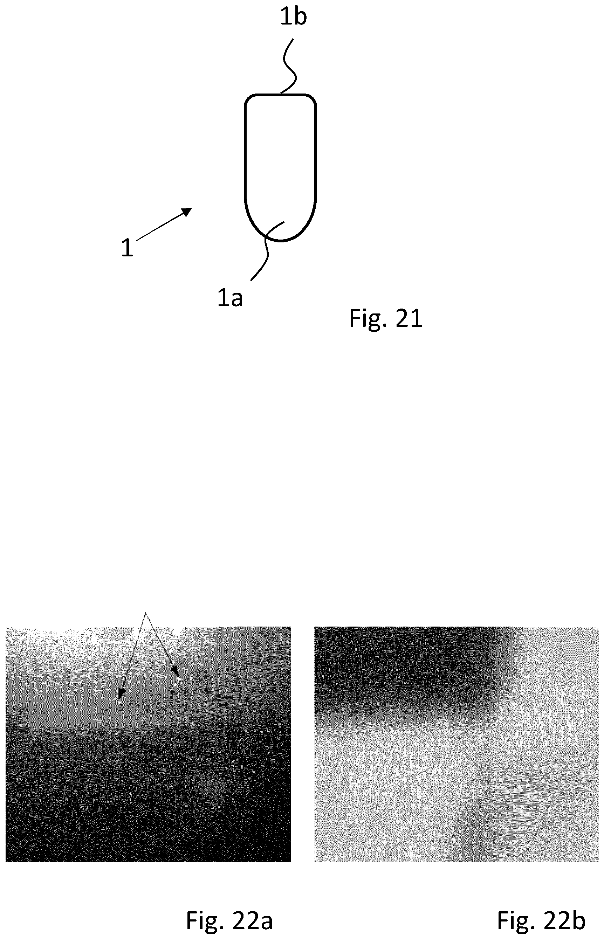

Fig. 21 shows an implant for stabilizing the wall of a alveolar channel.

Fig. 22a shows the surface of an etched implant in comparison with a laser polished and etched surface according to Fig. 22b.

### Detailed Description

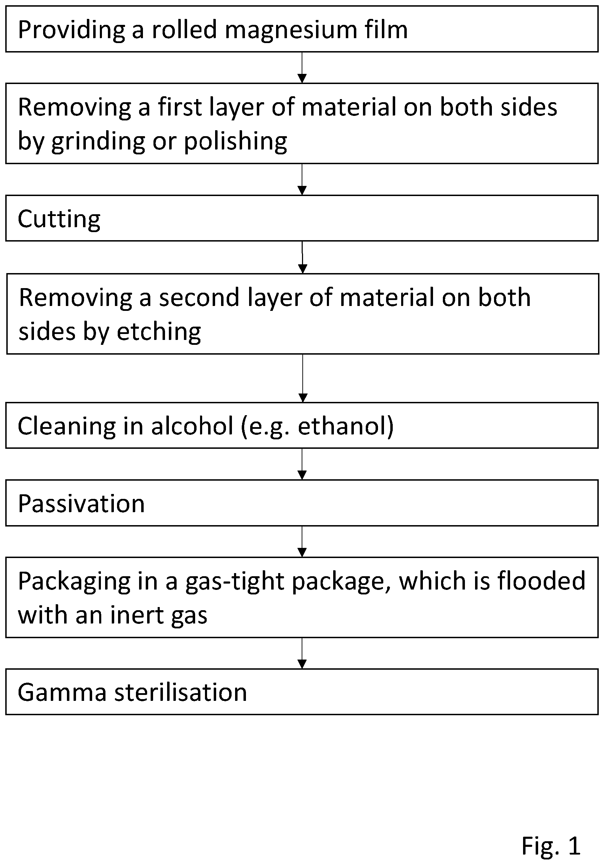

Fig. 1 is a flowchart of a method of producing an implant according to an embodiment of the disclosure.

As raw material, a rolled pure magnesium film is provided with a thickness to match the desired final thickness of the implant of 120 - 160 µm. A raw material having a thickness of 220 - 280 µm may be preferable for such a final implant thickness.

The raw material is inserted into a grinding or polishing machine and a first layer of material is removed on both sides. Now, the raw material has a thickness of 150 - 200 µm.

Then, the raw material is cut which defines the size and/or shape of the implant. In further the pieces can be shaped, in particular by bending.

Then, a second layer on each side of the implant is removed by immersing the implant into an etching bath.

Then, the implants are cleaned, preferably in alcohol.

Then, the implants are dried, preferably by storing the implants in a desiccator.

Then, the implants are passivated by storing the implants in a cabinet or clean room, preferably class 7 or better.

Aften a protective oxide film is formed on a surface, the implants are packed into a gas-tight foil bag, which is flooded with an inert gas.

Then, the package including the implant can be sterilized by using gamma radiation.

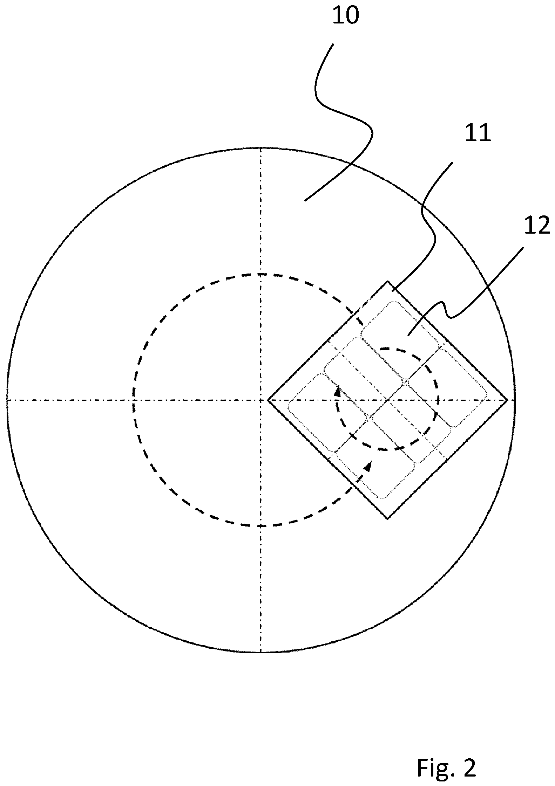

Fig. 2 shows schematically how the raw material 12 is grinded by using a horizonal grinding machine.

A magnesium foil is used as raw material 12, which is removably attached to a substrate holder 11.

The substrate holder 11 is eccentrically placed relative to the axis of a rotating grinding disk 10. While the grinding disk 10 rotated, the substrate holder is rotated 11 also.

The substrate holder 11 may comprise an adhesive surface onto which the raw material 12 sticks. Alternatively, the magnesium sheet 12 may be mounted on the substrate holder 11 by vacuum.

The grinding disk 10 has a grain size between 1000 and 2000 according to DIN ISO 6344-1 - 2000-04.

The grinding process is performed on both sides of the raw material and results in a roughness Ra below 0.2 µm, preferably below 0.1 µm.

Then, the raw material 12 is cut into pieces. According to an embodiment of the invention, a laser cutting process is used. Preferably, the implants can be cut in such a way that that the implants are still connected to the entire sheet by bridges. Before packaging, the implants can by separated by breaking the bridges.

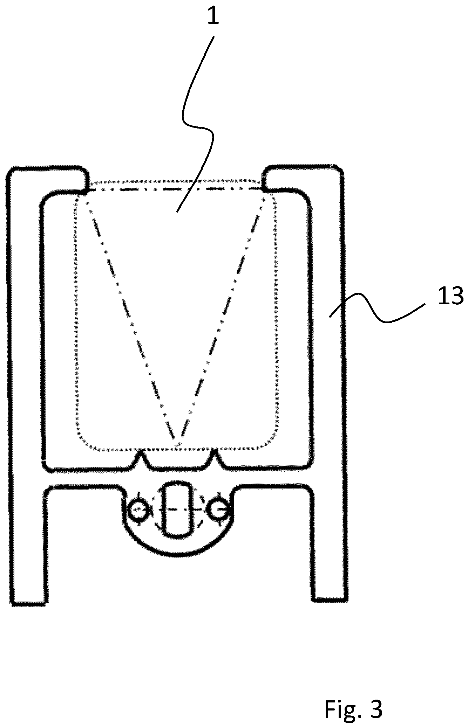

As shown in Fig. 3, stackable holders 13 are used for further processing of the implants 1.

The implants are inserted into the holders 13 and are spaced from each other in the stacked trays.

The holders 13 with the implants 1 are immersed into an etching bath.

The holders 13 are used also for the subsequent cleaning and drying of the implants 1.

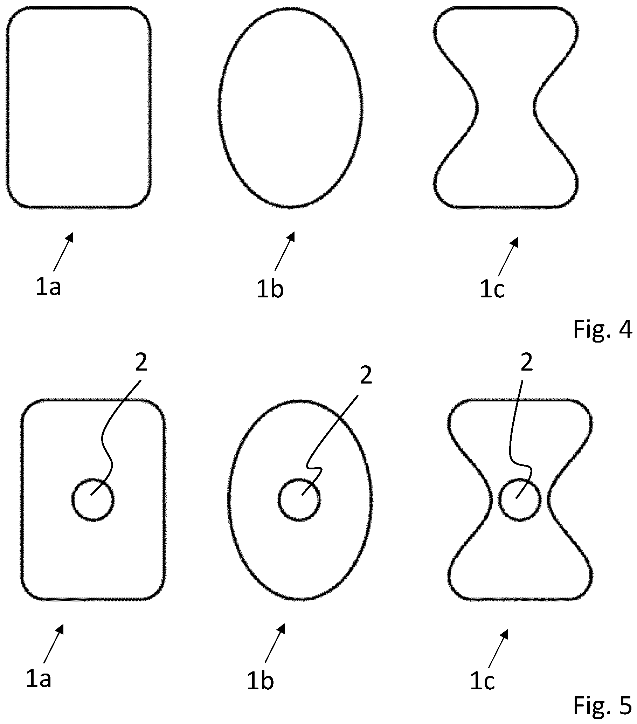

Fig. 4 shows three embodiments of an implant in a plan view.

The implant 1a- 1c consists of magnesium foil with a thickness of 100 - 200 µm. As shown, the implant can be embodied four-sided (1a), in particular rectangular with rounded edges, oval (1b) or the implant can have an hourglass-like shape (1c).

The embodiment with an hourglass shape 1c is preferably placed between two adjacent teeth, respectively tooth implants.

As shown in Figs. 5 all embodiments can have a central opening 2 for another implant, in particular for a tooth implant.

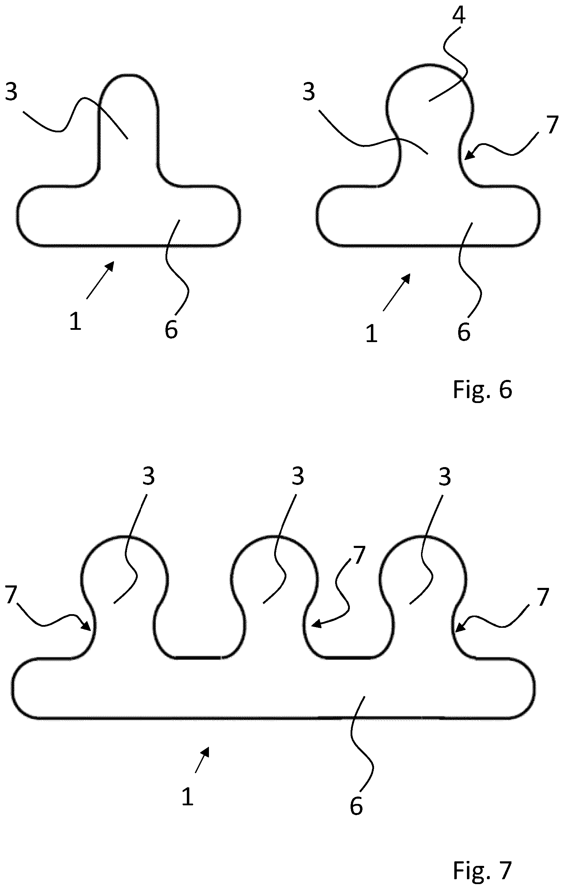

As shown in Fig. 6 the implant 1 can comprise at least one, in particular, exactly one finger-like extension 3 extending from the body 6 of the implant. The finger-like extension can comprise an area 4 with an increased width towards the end and a narrower neck-like portion 7 between the end 4 and the body 6. In particular, the finger-like extension 3 can comprise a circular shaped area 4.

As shown in a plan view according to Fig. 7, the implant 1 may comprise several, in particular 2-5 finger-like extensions 3. The teeth, especially tooth implants, can be placed between these finger-like extensions 3. The finger-like extensions 3 may pass through two existing teeth or a tooth implant may be placed between finger-like extensions 3.

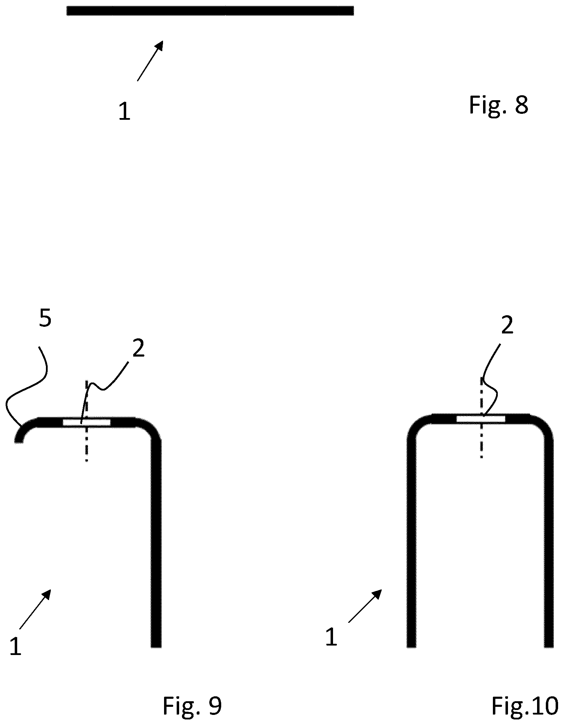

Fig. 8 - Fig. 10 are cross-sections of a various embodiments of an implant.

According to the embodiment of Fig. 8, the implant 1 is flat.

Fig. 9 shows an implant 1 with an L-shape cross-section and which comprises an optional central opening 2.

At least one shank of the L-shaped implant can have a rounded edge 5 in order to form a stopper.

Fig. 10 shows an implant 1 which is U-shaped. This embodiment also comprises a optional central opening 2.

With reference to Fig. 11 the relative material ratio curve shall be explained in more detail. The material ratio curve describes which portion of the area is "material" if a plane which is parallel to the mean plane of the surface intersects with a sample starting from the highest peak and moved into the material to the lowest valley.

The results can be displayed either as a histogram which shows the percentage of the material above and below a median plane of the surface structure or as the Abbott-Firestone curve respectively a material aera ratio curve. The more material is within a functionally derived core zone, the more homogenous the surface is.

Two horizontal lines in the surface Abbott-Firestone curve are drawn with a deviation of +/- 1 µm.

Thus divides the curve, in accordance to DIN EN ISO 21920, into three zones, namely a peak zone, a core zone and a valley zone.

The material volume per unit sampling area of each zone is actually the area enclosed beneath the surface Abbott-Firestone curve and the horizontal line of this zone. The void volume per unit sampling area of each zone is the area enclosed above the top horizontal line of this zone and above the surface Abbott-Firestone curve.

According to this embodiment more than 95% is placed in a range of +/- 1 µm.

A surface structure with such a small bandwidth essentially minimizes the risk of nonuniform corrosion in the initial phase after insertion which would be undesirable as local break-through corrosion spots would annihilate the intended barrier function of the membrane. The implant with such a specific surface structure has improved corrosion properties.

Fig. 12 and 13 show alternative embodiments of an implant with a perforation.

Fig. 12 shows an implant 1 with regular pattern of round holes. Fig. 13 shows an implant 1 with a regular pattern of curves with the shape of a boomerang.

The perforation helps to bend the implant 1 also to complex 3-dimensional structures. Preferably, these embodiments are used a support member for another bioresorbable membrane, e.g. a collagen membrane, a non-woven bioresorbable plastic material etc.

With reference to Fig. 14 - Fig. 17, embodiments of a method for inserting a dental implant are explained.

According to a first embodiment of the method, shown in Fig. 14, the magnesium or magnesium alloy foil 1 is clamped adjacent to a cavity 11 (defect side) between the soft tissue 10 and the bone.

The foil 1 is cut and shaped to the desired profile and stabilizes the buccal or palatal wall. Then, the cavity 11 is filled with a bone substitution material. As bone substitution material, a granular calcium phosphate material, can be used.

Optionally, the top side of the cavity 11 can be closes by using another membrane, e.g. a collagen membrane 13. Then, the defect side is closed with soft tissue (not shown).

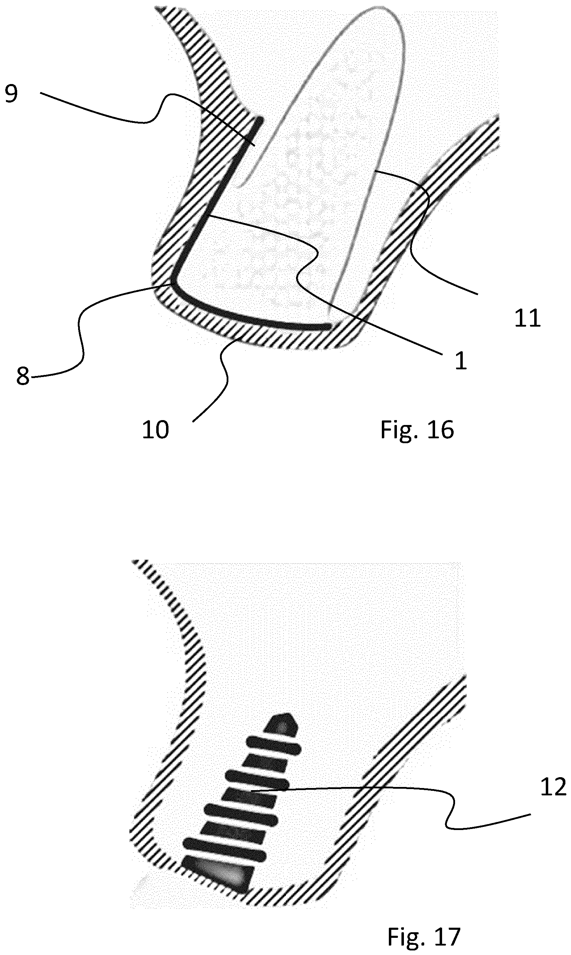

After healing of the defect side by forming natural bone tissue, a dental implant can be inserted (see Fig. 17).

Fig. 15/16 show another embodiment of a method for inserting a dental implant.

As shown in Fig. 15, the foil 1 is clamped between soft tissue 8 and bone 9 also.

As shown in Fig. 16, the foil 1 comprises a protrusion, which if folded above the cavity 11, which is filled with a bone substitution material. The folding edge 8, which is formed, stabilizes the three-dimensional shape of the foil 1.

After healing of the defect side, the dental implant 12, e.g. a screw for an artificial tooth, can be inserted, as shown in Fig. 17.

With reference to Fig. 14 - Fig. 17, embodiments of a method for inserting a dental implant are explained.

Fig. 18a and 18b illustrate the use of a magnesium or magnesium alloy film 1 for single wall defects. Fig. 18a is a perspective view and Fig. 18b is a top view.

The implant consisting of a foil 1 is bent to an appropriate three-dimensional form and closes the sidewall of the cavity 11. Then, the cavity is filled with the bone substitution material.

In this application, the foil may not be clamped between bone and soft tissues. Instead, a screw fixation using biodegradable Mg-screws allows adjustment and correction (not shown).

Fig. 19a and 19b illustrate the use of a magnesium or magnesium alloy film for double wall defects. In this case, the film 1 closes the buccal and palatal wall of the cavity 11.

Fig. 20 shows an embodiment of a magnesium or magnesium alloy implant which is folded to a convex bulge to increase the volume of the restored bone.

A bend smooth edge 15 prevents piercing of/cutting through the gingiva. The edge can also be stiffed by single or multiple bending the edge 15.

The foil 1 can also be folded to convex bulge 16 as shown in Fig. 20. Such a bulge 16, folded like a paper bag, can be inserted and placed onto the bone of a defect side.

Fig. 21 shows an implant for stabilizing the wall of a alveolar channel.

The implant being embodied as a magnesium or magnesium alloy film 1 has shield-like shape.

The film 1 comprises a rounded/acute tip 1a which can be pushed between bone and soft tissue, thereby fixing the foil by clamping only.

The top edge 1b of the film 1 is preferably substantially straight.

Fig. 22a shows the surface of an etched implant. Organic inclusions may result in spots (marked with arrows) on the surface after etching-. This may result due to a reduced corrosive attack during etching in the spot area. By laser polishing, as shown in Fig. 22b, such spots can be avoided.

## Claims

1. A method for producing an implant for covering bone defects, wherein the implant consists of a magnesium or magnesium alloy film, comprising:
providing a rolled magnesium or magnesium alloy film;
removing a layer of material on both sides of the magnesium or magnesium alloy film.

2. The method according to the preceding claim, wherein the layer of material is removed by grinding or polishing.

3. The method according to any of the preceding claims, wherein a thickness of the magnesium alloy film is reduced by 3 to 60 %.

4. The method according to any of the preceding claims, wherein a thickness of the magnesium or magnesium alloy film is reduced by a minimum of 10 µm on each side.

5. The method according to any of the preceding claims, wherein a remaining thickness of the magnesium or magnesium alloy film after grinding is further reduced on each side by 5 - 30 µm by etching.

6. The method according to any of the preceding claims, further comprising cutting or shaping the magnesium or magnesium alloy film between the grinding or polishing step and the etching step.

7. The method according to any of the preceding claims, wherein a magnesium or magnesium alloy film is produced with a material area ratio in a core zone of +/- 1 µm as determined from the Abbott-Firestone curve which is greater than 95%.

8. A Method for producing an implant for covering bone defects, in particular according to any of the preceding claims, wherein the implant consists of a magnesium or magnesium alloy film, comprising:
providing a rolled magnesium or magnesium alloy film;
laser treating a surface of the magnesium or magnesium alloy film.

9. The method according to the preceding claim, **characterized in that** the surface of the magnesium or magnesium alloy is laser-polished.

10. A Method for producing an implant for covering bone defects, in particular according to any of the preceding claims, wherein the implant consists of a magnesium or magnesium alloy film, comprising the step of purifying a surface of the magnesium or magnesium alloy film by mechanical means or by physical means.

11. An implant, being produced with a method according to any of the preceding claims.

12. Implant according to the preceding claim, wherein said implant is embodied as a convex bulge consisting of the magnesium or magnesium alloy film.

13. Implant according to any of the preceding claims, wherein the implant is folded.

14. Implant according to any of the preceding claims, wherein the magnesium or magnesium alloy film has a reduced valley depth Rvk of less than 0.6 µm, according to DIN EN ISO 21920 - Parts 1-3: 2021-12 and ISO 25178 - Parts 1-3;
and/or wherein the magnesium or magnesium alloy film has a maximum surface roughness Rt of less than 3 µm, according to DIN EN ISO 21920 - Parts 1-3: 2021-12 and ISO 25178 - Parts 1-3);
and/or wherein the film has a root mean square gradient Sdq of less than 5°, according to DIN EN ISO 21920 - Parts 1-3: 2021-12 and ISO 25178 - Parts 1-3;
and/or wherein the film has a thickness between 100 and 200 µm.

15. Implant according to any of the preceding claims, wherein the profile of the relative Material Ratio Curve ranges from - 2.0 µm to + 1.7 µηι, according to DIN EN ISO 21920 - Parts 1-3: 2021-12 and ISO 25178 - Parts 1-3.
